# EUROPEAN PATENT SPECIFICATION

(11) **EP 3 755 308 B1**
(45) Date of publication and mention of the grant of the patent: **16.02.2022**
(21) Application number: 19719927.6
(22) Date of filing: 22.02.2019
(51) Int. Cl.: A61K 9/16, A61K 31/4412, A61K 31/519, A61P 35/00

(54) **POROUS EMBOLIZATION MICROSPHERES COMPRISING DRUGS**
PORÖSE EMBOLISATIONSMIKROSPHÄREN MIT WIRKSTOFFEN
MICROSPHÈRES D'EMBOLISATION POREUSES COMPRENANT DES MÉDICAMENTS

(30) Priority: 23.02.2018 NL 2020487
(43) Date of publication of application: 30.12.2020
(73) Proprietor: van Rijn Beheer B.V., 3755 BT Eemnes (NL)
(72) Inventor: KOOLE, Levinus Hendrik, 6271 EC Gulpen (NL)
(74) Representative: EP&C
(86) International application number: PCT/NL2019/050116
(87) International publication number: WO 2019/164397

(56) References cited:
- WO-A1-2007/085615
- WO-A1-2007/090897
- WO-A1-2009/022190
- WO-A1-2012/101455
- US-A1- 2002 009 415
- US-A1- 2016 228 556
- YAN X ET AL: "Cisplatin delivery from poly(acrylic acid-co-methyl methacrylate) microparticles", JOURNAL OF CONTROLLED RELEASE, ELSEVIER, AMSTERDAM, NL, vol. 106, no. 1-2, 18 August 2005 (2005-08-18), pages 198-208, XP027664206, ISSN: 0168-3659 [retrieved on 2005-08-18]

## Description

### Technical Field

The current invention relates to a method of forming injectable polymeric microspheres loaded with therapeutic agent as claimed, to injectable polymeric microspheres loaded with therapeutic agent as claimed, to a kit comprising a container with injectable polymeric microspheres loaded with therapeutic agent as claimed, to a pharmaceutical composition comprising injectable polymeric microspheres loaded with therapeutic agent as claimed, to injectable polymeric microspheres loaded with therapeutic agent for use as a medicament as claimed, and to a pharmaceutical composition comprising injectable polymeric microspheres loaded with therapeutic agent for use as a medicament as claimed.

### Background Art

Embolization therapy is a well-established and trusted minimally invasive, image-guided technique for treating tumors. During embolization therapy a physician (interventional radiologist) uses embolic particles to block the blood flow to a tumor vascular bed. A catheter is inserted percutaneously and guided toward the tumor. Navigation is done under continuous X-ray fluoroscopy, and a contrast agent is used. There are various embolization methods, comprising: bland embolization, transarterial chemoembolization (TACE) and TACE with drug-eluting beads (DEB-DACE).

Bland embolization is also referred to as a first generation embolization technique. The objective of this therapy is solely to block capillary arteries feeding the tumor. In this procedure the tumor feeding arteries are catheterized and embolic particles are injected. The microparticles used in bland embolization are available in various shapes, sizes and materials. Spherical particles are preferred. Some examples of microspheres used for bland embolization are polyvinyl alcohol (PVA) microspheres, hydrogel PVA microspheres, super absorbent polymer microspheres (SAP-MS), and tris-acryl cross-linked gelatin microspheres (Embospheres^{®}, Merit Medical).

Transarterial chemoembolization (TACE) is a second generation embolization technique and also the most commonly used technique. In this method both chemotherapeutic agents as well as embolization materials are delivered to the tumorous tissue. During the TACE procedure a chemotherapeutic agent is injected into the tumor immediately followed by embolic microspheres. This induces high local drug concentration and stimulates prolonged retention of the drug in the tumor. The increased uptake of the chemotherapeutic agent causes the dosage delivered to the tumor to be several times above a lethal dose. This cannot be safely achieved with systemic chemotherapy.

DEB-TACE is a third-generation embolization technique. It is a single step embolization method in which embolic microspheres loaded with a drug are injected directly into the tumor vasculature enabling gradual release of therapeutics to the tumor bed in addition to vessel obstruction. DEB-TACE presents advantages over TACE in that the level of occlusion, amount of drug delivered to the site and duration of drug release at the site can be controlled.

The objective of DEB-TACE is to deliver drugs in a targeted manner with the microspheres serving as the drug delivery vehicle. This enables the microsphere to have a dual action; firstly they will obstruct the blood flow to the tumor and subsequently they will locally deliver the drug. Drug-eluting beads (DEBs) are usually loaded with a cytostatic such as doxorubicin. In DEB-TACE drugs are delivered in a precise, sustained and controlled manner. It decreases systemic release of the drugs whilst maintaining high intra-tumoral concentrations of a drug. Another advantage of using drug loaded microspheres is that the concentration of the chemotherapeutic agent in the body is minimal thus preventing the major side effects experienced during systemic chemotherapy.

However, polymeric microparticles which are used in current clinical practice in DEB-TACE procedures are still imperfect. For example, embolic microspheres for DEB-TACE which are available on the market, such as the DC bead^{®} (Biocompatibles UK, Ltd.), require that microspheres are first incubated in an aqueous solution of the drug. The beads are initially delivered to the interventional radiologist in the unloaded state. The drug is loaded onto the microspheres preceding the actual TACE procedure. This step is taken in the angiosuite. During incubation, adsorption of the drug onto the polymer structure (containing negatively charged sulfonate groups) takes place. Thus, the drug-loaded microspheres have to be prepared prior to the procedure to allow the microspheres to pick up drug. This means that the embolization procedure cannot commence before the drug loading process is complete (approximately 30 min). This is cost-enhancing and therefore a disadvantage. Another disadvantage is that the interventional radiologist performing the procedure has to work with pure cytostatics as he or she needs to mix the embolisation particles and the cytostatics. This is not preferred due to the toxicity of cytostatics.

Furthermore, prior art beads such as the DC beads^{®} are only covered by a layer of drug, with only little penetration of the drug into the bead. When injected *in vivo,* drug release from the microspheres will be very high in the initial phase of the procedure - potentially even when the microspheres are not yet at the tumor site - decreasing the amount available for prolonged exposure of drug to the tumor. Similar observations are made for beads disclosed in US2016/228556, WO2007/090897, WO2007/085615, WO2007/022190, WO2012/101455, US2002/009415 or in Yan et al. J. Controlled Release, 2005-08-18, 106, 1-2, 198-208. The beads or spheres are swollen and impregnated with the drug. This typically leads to a high initial burst release of therapeutic agents deposited at the outside and a relative low and short-lasting sustained release profile.

Moreover, during incubation of the DC beads^{®}, adsorption of the drug onto the polymer structure which contains negatively charged sulfonate groups takes place. Not surprisingly, drug binding is most efficient for drugs that carry positively charged groups in their molecular structure. Drugs which are not charged or negatively charged can hardly be loaded onto the mentioned prior art beads, which is another disadvantage.

Other drug eluting beads have recently been developed. *Porous* embolization microspheres, such as those disclosed in WO 2009/086098 for example, can also contain drugs inside of the microspheres, rather than only on the outer layer such as the DC beads^{®}, which theoretically increases the amount of drugs that can be loaded onto and into the drug eluting beads. However, in practice, the embolization microspheres are still loaded by immersing the microspheres in a solution of drugs in water. The solution of drugs and water enters the pores of the microspheres, thereby loading the porous microspheres with the drugs. Most cytostatics are however poorly soluble in water. The inherently low concentration of the drugs in water leads to a low amount of drugs in the pores of the microspheres.

### Summary of Invention

The invention is defined by the claims. Any subject-matter falling outside the scope of the claims is provided for information purposes only.

The present invention aims to overcome one or more of the abovementioned drawbacks, or at least to provide a useful alternative. Thereto, the current invention provides a method of forming injectable polymeric microspheres loaded with therapeutic agent, comprising:
a. exposing porous polymeric microspheres to an organic solvent comprising a dissolved therapeutic agent thereby creating microspheres loaded with therapeutic agent,
b. separating the microspheres loaded with therapeutic agent from the organic solvent,
c. washing the microspheres loaded with therapeutic agent with water, and
d. drying the washed microspheres, preferably wherein the porous polymeric microspheres have pores having a size of between 2 and 50 micrometers, more preferably between 3 and 8 micrometers, even more preferably between 5 and 10 micrometers.

The method of the invention results in injectable polymeric microspheres loaded with therapeutic agent. The loading mechanism is particularly useful for drugs (in other words: therapeutic agents) featuring poor solubility in aqueous media and excellent solubility in organic media. The latter is a requirement that is met by the majority of the modern high-potent cytostatic and anti-angiogenic drugs. Moreover, contrary to the prior art (e.g. DC beads^{®}) the loading mechanism is not only effective for positively charged drugs. In fact, the drugs used for DC beads^{®} need to have a well-balanced positive charge in order to reach the optimum between loading capacity and release properties. The method of the present invention on the other hand works well for drug molecules with positive charges, negative charges, as well as neutral molecules, and is therefore more versatile. The method according to the present invention can therefore be used for personalized therapy, wherein the method is used for loading microspheres with medicament suitable for treatment of the particular disease of an individual.

Incubation of the porous 3D crosslinked microspheres in a concentrated solution of one, two or more drugs in organic solvent will lead to influx of the drugs and solvent in the porous structure of the microspheres, preferably as the microspheres adopt a swollen state. As the solvent is absorbed by the polymer network, there is concomitant transport of the drugs into the interior of the microspheres. After achievement of the equilibrium swollen state (typically after 4-6 h), the supernatant solution is carefully removed, and a volume of water (for example a water volume of about 10 times the volume of the microspheres) is added in order to wash the microspheres. The volume of water effectively extracts the solvent molecules out of the microspheres, thus inducing rapid precipitation of the drug(s), inside the microspheres. This takes advantage of the poor solubility of most cytostatic and anti-angiogenic drugs in water, as well as from much slower diffusion coefficients of the drug molecules as compared to solvent molecules. The washing step can be repeated, e.g. two, three or more times to remove effectively all solvent.

The consequence of the abovementioned treatment is that solid drug is deposited inside the microsphere pores, as well as on the surface of the microspheres. In this manner, a much higher degree of loading is achieved, in comparison with existing and commercially available embolization products, utilizing drug-loaded/drug-eluting embolic microspheres, which are loaded in water and are non-porous, and thus only comprise a thin layer of medicament on the surface of the particle.

During washing, the solvent is rapidly extracted from the microspheres due to its solubility in water, and outward diffusion of the solvent (being a small molecule) is fast. The consequences of the rapid departure of solvent from the swollen microsphere are: (i) rapid shrinking of the microsphere, approximately back to its original dimensions, and (ii), crystallization of the drugs inside the pores, as well as on the microsphere's surface. Drug crystals at the microsphere's surface may be physically connected to crystals inside the peripheral pores (vide infra). This results in stable binding of the "outside" drug crystals. Thus, the drug-loaded microspheres contain solid drug, in the pores and at the surface. The drug domains at the surface may be "interlocked" with drug crystals inside the pores.

The mechanism of in situ drug release, when embolic microspheres according to the invention are used will initially be primarily determined by the solubility of the drugs, and not so much by diffusion. The drug(s) is (are) available immediately at the surface of the embolic microspheres, so the diffusion pathway is relatively short. Only afterwards, when there is no drug left at the surface, drug release becomes dependent on diffusion, i.e. transport of drugs from the microsphere's interior to the surface, and subsequent release. This dual mechanism, also, contrasts with the drug release mechanism that governs in situ drug release from existing and competing commercial drug-eluting microspheres such as DC Beads^{®}. Its relevance for in situ tumor treatment is hypothesized to be large, as the dual mechanism allows for detailed engineering of the release kinetics, aimed at the realization of a therapeutic window of maximum duration.

The method according to the invention comprises a step of drying the separated microspheres. Such drying may be performed by leaving the microspheres out in open air, i.e. an ambient environment, until all water and any remaining solvent, if present, has evaporated. Evaporation may be assisted by exposing the microspheres to reduced pressure. Evaporation may be further assisted by slight heating of the microspheres, yet preferably not above 40 °C, more preferably not above 30 °C in order to prevent degradation of the drugs and/or the microspheres. Another option is freeze-drying of the microspheres. Due to the drying step, the method according to the invention results in dried microspheres which are pre-loaded with medication. This means that the microspheres can be delivered to the interventional radiologist in a state in which one or more drugs are present inside the pores and at the periphery of the microspheres. The only thing or things the medical practitioner has to do is: (i), suspend the drug-loaded microspheres in a physiological salt solution; and optionally: (ii) mix the suspension with liquid contrast agent, such as, for instance, Omnipaque 350^{®}. This makes the microspheres easier and safer to use than prior art injectable particles for DEB-TACE. In other words, the method according to the invention makes it possible to pre-load embolic microspheres. Microspheres can be loaded with drugs in a production facility, thereby reducing the amount of time needed for performing the embolization procedure and also reducing the risk for the interventional radiologist as working with the pure cytostatics is not necessary.

Although drying of conventionally used embolic particles such as the DC beads^{®} in their loaded state would theoretically be possible, resuspension of the embolic particles would lead to a significant loss of the drug due to the solubility of the positively charged drug in water and due to the medication only being present at the surface of the particles. Such resuspension of the prior art particles would lower the already low amount of drug on the beads, which makes DC beads^{®} and comparable particles unattractive for preparing ready-to-use particles, i.e. particles which do not have to be loaded immediately before the embolization procedure.

In the method according to the invention, a higher absolute loading is achieved, i.e. microspheres loaded with the method according to the invention comprise a substantially higher amount of drugs as compared to prior art particles. Therefore, although upon resuspension of the microspheres according to the present invention some drugs may be released from the microspheres, the absolute amount of drugs on the microspheres will remain much higher as compared to conventional particles on which the method according to the invention would mutatis mutandis be used. Especially in the case of drugs which are poorly soluble in water, any loss of drugs upon resuspension in water will be little. On the contrary, many prior art particles cannot even be used with drugs that are poorly soluble in water.

The drug-loaded embolic microspheres according to the invention effectively combine embolization properties, local drug delivery and enhanced drug capacity. This translates into prolonged therapeutic concentrations, i.e. an expanded therapeutic window. The drug-loaded microspheres according to this invention are also easier to use for a medical practitioner than conventional embolic particles.

The microspheres according to the invention are substantially spherical. As used herein, "substantially spherical" generally means a shape that is close to a perfect sphere, which is defined as a volume that presents the lowest external surface area. Specifically, "substantially spherical" in the present invention means, when viewing any cross-section of the particle, that the difference between the major diameter and the minor diameter is less than 20% of the major diameter. The advantage of spherical particles is twofold: (i), spherical particles have a maximum tendency towards solitary behaviour (i.e. the risk for clogging of microparticles is minimized) and (ii) better prediction of the position in the vessel tree where the particles will end up is possible. For non-spherical particles, the aspect ratio represents an uncertainty as to where the blood vessel diameter (which decreases upon entering the vascular tree inside tumor) and the size of the particle will match.

The invention additionally provides the injectable polymeric microspheres loaded with therapeutic agent of claim 11 obtainable by the method according to the invention.

The invention also provides the kit of claim 12 comprising a container with injectable polymeric microspheres loaded with therapeutic agent according to the invention.

The invention further comprises the pharmaceutical composition of claim 13 comprising injectable polymeric microspheres loaded with therapeutic agent according to the invention.

Finally, the invention relates to the injectable polymeric microspheres according to claim 11 for use as a medicament, preferably for the embolization treatment of tumours, and to the pharmaceutical composition according to claim 13 for use as a medicament, preferably for the embolization treatment of tumours.

### Description of Embodiments

The full advantages of the invention are achieved when the organic solvent is at least partially miscible with water at 20 °C. Preferably, the organic solvent is fully miscible with water at 20 °C. This leads to fast diffusion of solvent molecules out of the microspheres in step c. of the method according to the invention as well as to optimal retention of the therapeutic agent or agents in the microspheres during washing and thus to a higher loading of the microspheres.

The concentration of the therapeutic agent in the organic solvent preferably is at least 20 mg/mL. Such a concentration leads to injectable polymeric microspheres comprising a high amount of therapeutic agent, such as for example at least 10 w% of therapeutic agent with respect to the weight of the loaded microsphere.

More preferably, the concentration of the therapeutic agent in the organic solvent is at least 100 mg/mL at 20 °C. Such a concentration leads to injectable polymeric microspheres comprising a particularly high amount of therapeutic agent, such as for example at least 12 w% of therapeutic agent with respect to the weight of the loaded microsphere. Most preferably, the concentration of the therapeutic agent in the organic solvent is at least 200 mg/ mL at 20 °C. Such a concentration leads to an optimal loading degree of the microspheres, such as at least 14 w% of therapeutic agent with respect to the weight of the loaded microsphere.

Preferably, the concentration of the therapeutic agent in the organic solvent is at most 800 mg/mL at 20 °C. This keeps loss of medicament which is not loaded onto the microspheres at an acceptable level. More preferably, the concentration of the therapeutic agent in the organic solvent is at most 500 mg/mL at 20 °C, as this results in high loading of the microspheres and an acceptable amount of loss of unloaded dissolved therapeutic agent.

The solubility of the therapeutic agent in water is preferably between 5 and 1000 mg/L at 20 °C. At lower solubilities, release kinetics in the body of a patient are too slow for practical use. At higher solubilities, too many drug molecules are released from the microspheres during washing step c. and/or during resuspension of the drugs before use.

More preferably, the solubility of the therapeutic agent in water is between 5 and 100 mg/L at 20 °C. At lower solubilities, release kinetics in the body of a patient are too slow for practical use. At higher solubilities, a relatively high amount of therapeutic agent is released from the microspheres during washing step c. and/or during resuspension of the drugs before use. Most preferably, the solubility of the therapeutic agent in water is between 10 and 50 mg/L, as this solubility range provides an optimum between release kinetics and loading efficiency.

Preferably, in step a. the porous polymeric microspheres swell in the organic solvent. As the microspheres swell, so do the pores of the microspheres, which increases the capacity of drug uptake as compared to microspheres which are not swollen.

Preferably the porous polymeric microspheres are swellable in the organic solvent to about 200 to 1000 % of their dry size, more preferably to about 500 to 1000 %, most preferably to about 800 to 1000 %. A high swelling degree of the microspheres in the organic solvent results in an increased amount of drug in the pores of the microspheres.

In another preferred embodiment the porous polymeric microspheres are swellable in the organic solvent to a swelling ratio Q, defined by the weight of the swollen particles divided by the weight of the dry particles, of about 1 - 200, preferably of 2 - 20, more preferably of 5 - 10. A high swelling ratio Q of the microspheres in the organic solvent results in an increased amount of drug in the pores of the microspheres.

Preferably in step a. a reduced pressure, more preferably a vacuum is applied. The vacuum is applied while the microspheres are submerged in the drug solution. At low pressure air bubbles, present in the interior of the microspheres, will expand and escape from the microspheres. Removal of air bubbles will add to the capacity of the porous microspheres, and therefore in the drug loading procedure the reduced pressure treatment is advantageous.

Preferably the solution comprising an organic solvent and a dissolved therapeutic agent comprises multiple therapeutic agents. This will create injectable polymeric microspheres loaded with multiple therapeutic agents. Such microspheres are suitable for combining multiple treatment strategies at the same time. Because loading of the microspheres is not dependent on ionic interactions, there will be no or only limited competing interactions between the different therapeutic agents. Loading the microspheres with multiple therapeutic agents is therefore mainly dependent on the relative amount of the therapeutic agents in the solution. This provides for a simple adjustment method of the relative amounts of both therapeutic agents.

In a preferred embodiment the solution consists of the organic solvent and one or more therapeutic agents. In an alternative preferred embodiment, the solution comprises a mixture of organic solvents and one or more therapeutic agents. A combination of different organic solvents may lead to increased solubility of the therapeutic agents.

In a preferred embodiment, the organic solvent is chosen from the group consisting of acetaldehyde, acetic acid, acetone, acetonitrile, 1,2-butanediol, 1,3-butanediol, 1,4-butanediol, 2-botoxyethanol, butyric acid, diethanolamine, diethylenetriamine, dimethylformamide, dimethoxyethane, dimethyl sulfoxide, 1,4-dioxane, ethanol, ethylamine, ethylene glycol, formic acid, furfuryl alcohol, glycerol, methanol, methyl diethanolamine, methyl isocyanide, N-methyl-2-pyrrolidinone, 1-propanol, 1,3-propanediol, 1,5-pentanediol, 2-propanol, propanoic acid, propylene glycol, pyridine, tetrahydrofuran, triethylene glycol, hexamethylphosphoramide, and/or mixtures thereof.

More preferably the organic solvent is chosen from the group consisting of dimethyl sulfoxide (DMSO), hexamethylphosphoramide (HMPA), dimethylformamide (DMF) and water soluble alcohols such as 1,2-butanediol, 1,3-butanediol, 1,4-butanediol, 2-butoxyethanol, ethanol, (ethylene) glycol, furfuryl alcohol, glycerol, methanol, 1-propanol, 1,3-propanediol, 1,5-pentanediol, 2-propanol, and/or mixtures thereof.

Most preferably, the organic solvent is DMSO. DMSO is a pharmaceutically acceptable solvent with relatively low toxicity compared to many other organic solvents. Furthermore, many anticancer drugs are particularly soluble in DMSO, making DMSO a versatile solvent which can be used in a method according to the invention for a wide range of drugs. Furthermore, DMSO is miscible with water, such that in step c. the outward diffusion of DMSO is particularly fast, resulting in a particularly high drug loading capacity of the microspheres. Organic solvents, notably DMSO, are easily imbibed into the 3D-crosslinked network structures of the porous polymeric microspheres. In other words: the 3D crosslinked microspheres may show considerable swelling (such as 8 - 10 times volume-wise) upon incubation in DMSO.

Preferably the therapeutic agent is an anti-cancer drug. The injectable polymeric microspheres according to the present invention are particularly useful for embolization therapy, which in its turn is an effective method for treating cancer tumors. Incorporation of anti-cancer drugs in microspheres for embolization increases the effect of the embolization treatment.

Another preferable class of therapeutic drugs comprises anti-angiogenic drugs. These medicaments retard or inhibit the formation of new blood vessels (angiogenesis). Angiogenesis is controlled by chemical signals which can stimulate both the repair of damaged blood vessels and the formation of new blood vessels. Normally, the stimulating and inhibiting effects of these chemical signals are balanced so that blood vessels form only when and where they are needed. Angiogenesis plays a critical role in the growth and spread of cancer, certainly in cases of embolization in which parts of the tumor become suddenly deprived of oxygen supply since the influx of blood is blocked. Tumors can stimulate nearby normal cells to produce angiogenesis signaling molecules. Angiogenesis inhibitors interfere with various steps in this process. For example, the drugs sorafenib and sunitinib bind to receptors on the surface of endothelial cells or to other proteins in the downstream signaling pathways, blocking their activities. Another prominent angiogenesis inhibitor is bevacizumab (Avastin^{®}), a monoclonal antibody that specifically recognizes and binds to and blocks vascular endothelial growth factor.

Preferably, the therapeutic agent is chosen from the group consisting of sorafenib, irinotecan, cis-platin, paclitaxel, docetaxel, cabazitaxel, larotaxel, eribulin, ixabepilone, vinflumine, peretinoin, orantinib, brivanib, sunitinib, briganib, erlotinib, lenvatinib, crizotinib, and vandetanib. These drugs have a low solubility in water, yet a good solubility in organic solvents, notably DMSO. The method according to the invention is particularly suitable for loading embolization microspheres with these therapeutic agents.

More preferably, the therapeutic agent is chosen from the group consisting of sorafenib, paclitaxel, docetaxel, cabazitaxel, larotaxel, eribulin, ixabepilone, peretinoin, orantinib, brivanib, sunitinib, briganib, erlotinib, lenvatinib, crizotinib, and/or vandetanib. These drugs have a low solubility in water, yet a good solubility in organic solvents, notably DMSO. The method according to the invention is most particularly suitable for loading embolization microspheres with these therapeutic agents.

Preferably, the anti-cancer drug is sorafenib.

Preferably, the polymeric microspheres are internally cross-linked. Crosslinking of the polymeric microspheres is achieved during synthesis (i.e., during the polymerization process) of the polymeric microspheres through the use of a cross-linking agent in the cocktail of starting materials (reactive monomers). Preferably, the crosslinking agent is a diacrylate or di-methacrylate structure, such as ethyleneglycoldimethacrylate (EGDMA), diethyleneglycoldimethacrylate (DEGDMA), triethyleneglycoldimethacrylate (TEGDMA), ethyleneglycoldiacrylate (EGDA), diethyleneglycoldiacrylate (DEGDA), or triethyleneglycoldiacrylate (TEGDA). More preferably, the crosslinker is triethyleneglycoldimethacrylate (TEGDMA).

Preferably, the porous polymeric microspheres comprise between 5 and 30, more preferably 10 - 20 weight % of TEGDMA based on the total weight of the porous polymeric microspheres.

Preferably, the porous polymeric microspheres comprise methacrylic monomer units, such as methyl methacrylate (MMA). More preferably, the porous polymeric microspheres comprise between 20 and 60 w% of MMA, more preferably between 30 and 50 w% of MMA.

Hydrophilicity of the porous polymeric microspheres is preferred to avoid clustering of the microspheres before or during injection of the microspheres in a patient. Thereto, the polymeric microspheres preferably comprise hydrophilic methacrylic monomer units, such as hydroxyethyl methacrylate (HEMA). Preferably, the porous polymeric microspheres comprise between 20 and 60 w% of HEMA, more preferably between 30 and 50 w% of HEMA.

Preferably, the porous polymeric microspheres are radiopaque. Neither the DC beads^{®} nor the beads disclosed in WO 2009/086098 are intrinsically radiopaque, creating limitations because their location in the body cannot be determined. If visibility is required, a radiopaque agent can be injected into the arteries immediately before or after injecting the polymeric microspheres. This gives an indication of the position of the microspheres, but not their precise location. One cannot be sure that the microspheres did not unintentionally leak into healthy tissue (a phenomenon known in the professional domain of interventional radiology as "reflux"). In order to prevent such problems, the microspheres themselves can be loaded with a radiopaque agent. This leads to a more precise localization of the microspheres, but reduces the amount of space in the particle that is available for the drug, thus decreasing the effective drug-loading capacity of the microspheres. Preferably, the porous polymeric microspheres comprise iodine. Such intrinsically radiopaque porous polymeric microspheres may for example be synthesized by incorporation of an iodine containing monomer, preferably the iodine containing monomer 2-[4-iodobenzoyloxy]-ethyl methacrylate (4IEMA).

In a most preferred embodiment, the porous polymeric microspheres comprise a copolymer of between 10 and 20 w% of 4IEMA, between 30 and 50 w% of MMA, between 30 and 50 w% of HEMA, and between 10 and 20 w% of crosslinker TEGDMA, the total adding up to 100w%. Such microspheres have an optimum performance in the method of the present invention. Such microspheres do not swell more than to 200 % of their dry size in a physiological fluid and have an optimal balance between hydrophilicity, swellability in organic solvent, radiopacity, and shape-retainment under shear in physiological conditions.

In order to obtain porosity, during the synthesis of the microspheres, the monomer mixture can be supplemented with removable particles. The removable (solid) particles are incorporated in the polymeric microspheres. Subsequent dissolution of the particles leads to dissolution (leaching out) of the removable particles resulting in porous polymeric microspheres. Typical and preferred examples are PPMA and silver.

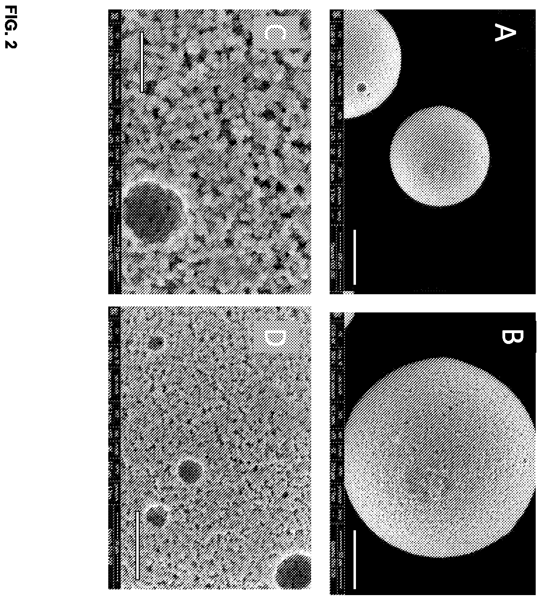

For instance, a method for the preparation of polymeric microspheres contains a step in which polymethylmethacrylate (PMMA) dissolved in toluene is added to the monomers and polymer that will make up the microsphere. After the synthesis, the PMMA can be dissolved (i.e. eluted) from the solid microspheres, resulting in microporosity by the generation of pores (see for instance Fig 2C and D). Preferably, the pores or cavities of the polymeric microspheres have a size of between 2 and 50 micrometers. More preferably between 5 and 10 micrometers. A "porous particle" is a particle that contains pores, which may be observed and determined, for example, by viewing the microspheres using a suitable microscopy technique such as scanning electron microscopy. Pore size may vary widely, ranging from 0.5 micron or less to 1 to 2 microns to 5 microns to 10 microns to 25 microns to 50 microns to 100 microns or more. Pores can come in a wide range of shapes and thus need not be cylindrical. In some embodiments, the particles comprise a porous surface layer disposed over a non-porous core. In other embodiments, pores are present throughout the interior of the particles.

Additionally or alternatively, the monomer mixture can be supplemented with silver particles. These particles can be dissolved by nitric acid, resulting in the formation of pores (microporosity). This has a significant advantage, since the pores that are obtained are essentially envelopes of the embedded silver particles. This implies that the size of the pores can be engineered through the choice of the metallic particles: larger metallic particles will produce larger pores, and smaller metallic pores will produce smaller pores. While the provision of porosity in the microspheres with the inclusion of PMMA and subsequent elution by a solvent provides good results, also in terms of loading with a therapeutic agent, the control over the pore size and pore size distribution is less. There is the inherent risk that the pores formed may be too small to be effectively loaded or that the pore size distribution is unfavourable to allow for a high load of therapeutic agent or the release profile is less well controllable. Therefor, in a preferred embodiment, there is a preference for the incorporation of solid particles in the generation of the microspheres. By the inclusion of solid particles in the microspheres, the desired pore size and pore size distribution is more controllable and hence the subsequent load and release profile of the therapeutic agent from the porous microsphere. The solid particle is preferably a particle from a material that does not provoke any adverse reaction when the porous microsphere is used in a mammal. The material for the solid particle is preferably elutable from the microsphere, typically in a solvent that for the microsphere itself is inert. Preferably the material of the particle is independently selected from amongst silver, iron, non-noble metals (e.g. aluminium, zinc, magnesium, copper, tin and mixtures thereof), ceramics, calcium carbonate, calcium sulphate, preferably silver, non-noble metals and/or calcium carbonate. Once the microspheres have been formed, the solid particle can be dissolved or eluted from the microspheres. Dissolution or elution for the solid particles can be achieved by a suitable solvent such as an acid. Alternatively, solid polymer particles can be incorporated and subsequently eluted by a suitable (organic) solvent. Thus, PMMA particles (rather than a solution of PMMA) incorporated in the monomer/polymer mixture when forming the microspheres and subsequent leaching out using a solvent for PMMA such as toluene is also an embodiment of the invention.

The technical advantage residing in the dissolution of the solid particle from amongst the formed microspheres is an improved control over pore size and pore distribution. This leads in turn to an improved control over loading and the release rate of the therapeutic agent. The pores of the porous microspheres, preferably obtained by leaching out of solid particles, are preferable in the range of 1-5 micron, preferably 2-5 micron, with a preference for 2,5-3,5 micron. Preferably at least 80%, more preferably at least 85, even more preferably at least 90, and most preferably at least 95% of the pores of the porous microspheres have pores that are in these ranges. Larger pore sizes, although technically possible, are less preferred as the larger pore sizes tend to destabilize the microspheres that may have diameters in the preferred ranges of 50-100 micron which may lead to undesired fragmentation during handing.

Preferably, the pores are distributed throughout the entire volume of the porous polymeric microspheres. This leads to an optimal drug-loading efficiency.

The porous microspheres of the invention can also be loaded using a vacuum.

This allows achievement of higher loads of therapeutic agent.

In this embodiment, a step is introduced wherein the microspheres are subjected to reduced pressure (i.e. 0,5 bar, preferably 0,3 bar, more preferably 0,1 bar).

This reduction of pressure extracts air from the pores and subsequent impregnation of the porous microspheres with solutions containing therapeutic agent will also lead to higher loads of therapeutic agent. Subsequent contact of the impregnating solution containing the therapeutic agent with a non-solvent for the therapeutic (such as water) or drying will cause the therapeutic drug to deposit/crystallize in the pores.

Preferably, the injectable polymeric microspheres have a diameter in the range of from 1 - 1000 µm for a good embolic action, preferably of from 1 - 200 µm for better embolic action, more preferably of from 50 - 100 µm for an optimal embolic action.

Preferably, the injectable polymeric microspheres do not swell to more than 400 % of their dry size in a physiological fluid. Microspheres that swell to more than 400 % of their dry size in a physiological environment such as the bloodstream or the injection medium may deform severely under the shear forces that are exerted on the microspheres upon injection. Such deformation may cause the microspheres to release the therapeutic agent before arriving at the site to be treated, notably a tumor site. More preferably the injectable polymeric microspheres do not swell to more than 200 % of their dry size in a physiological fluid in order to avoid any substantial deformation. Most preferably, the polymeric microspheres do not swell in a physiological fluid.

Preferably, the injectable polymeric microspheres loaded with therapeutic agent comprise at least 10 weight%, preferably at least 12 weight%, more preferably at least 14 weight% of therapeutic agent with respect to the weight of the unloaded particle. A higher loading capacity leads to a more effective treatment upon use of the microspheres for DEB-TACE.

In certain embodiments, the porous microspheres of the invention can be loaded with relative high loads of therapeutic agents compared to microspheres that are not additionally equipped with pores or cavities, but instead have to rely on conventional swelling and inclusion behaviors. In certain embodiments the porous microspheres of the invention are loaded with a therapeutic agent to more than 200%, more than 300%, more than 400% compared to the microspheres prepared with the same chemical components and process, but without the leaching of the pore-forming (i.e. removable) substances (for instance PMMA or silver as described above), thus without pores or cavities.

In embodiments of the invention, the injectable polymeric microspheres comprise crystals of therapeutic agent on the outer surface of the microspheres interconnected with crystals of therapeutic agent inside the pores. This results in a minimum loss of therapeutic agent.

In the kit according to the invention, the injectable polymeric microspheres are in a dry (i.e. water and/or solvent free) state. Therefore, the injectable polymeric microspheres can be stored for long (e.g. 1 year) time periods without degradation of the therapeutic agent and/or reducing the amount of therapeutic agent in the microspheres.

The kit further comprises a container with a pharmaceutically injectable liquid. In this case, all the medical practitioner has to do immediately before the treatment is to (i), suspend the microspheres in the pharmaceutically injectable liquid, such as a physiological salt solution (0.9 % NaCl), and optionally (ii) mix the suspension with a commercially available contrast agent (such as, for instance, Omnipaque 350). In a preferred embodiment, the kit further comprises a container with contrast agent.

In a preferred embodiment, the injectable microspheres loaded with therapeutic agent are localized with X-ray spectroscopy. During the embolization therapy the physician (interventional radiologist) uses embolic microspheres to block blood flow to the tumor vascular bed. A catheter is inserted percutaneously into the femoral artery and guided toward the tumor. Navigation is done under continuous X-ray fluoroscopy, and a contrast agent is used.

The injectable polymeric microspheres according to the invention are useable as a medicament, preferably an injectable medicament, more preferably for the treatment of cancer, preferably liver cancer, more preferably hepatocellular carcinoma (HCC). Liver cancer poses a large worldwide burden, it is the 5^{th} most diagnosed cancer in men and 9^{th} most diagnosed cancer in women. It is the second most common cause of death from cancer globally, accounting for around 746,000 deaths in 2012. (1) Hepatocellular carcinoma (HCC) accounts for 70-85% of the liver cancer burden worldwide. (2) HCC is often diagnosed at an advanced stage, preventing curative treatments such as tumor ablation, partial hepatectomy or liver transplantation. A possible treatment option is embolization therapy. A catheter is inserted percutaneously into the femoral artery, through a small incision in the groin and guided toward the tumor. The efficacy of embolization for HCC relies heavily on the unusual morphology of the tumor that receives 80% of its blood supply from the hepatic artery, in contrast to the non-tumorous liver parenchyma which receive their blood supply from the portal vein. The advantage of the difference in blood supply to the tumor and healthy liver parenchyma creates a natural channel for targeted therapy, making embolization therapy particularly effective in the treatment of liver cancer, in particular HCC.

A pharmaceutical composition according to the invention preferably comprises one or more of a pharmaceutically acceptable diluent, vehicle, and/or excipient.

As required, detailed embodiments of the present invention are disclosed herein; however, it is to be understood that the disclosed embodiments are merely exemplary of the invention.

Therefore, specific structural and functional details disclosed herein are not to be interpreted as limiting, but merely as a basis for the claims and as a representative basis for teaching one skilled in the art to variously employ the present invention in any appropriately detailed structure. Further, the terms and phrases used herein are not intended to be limiting, but rather, to provide an understandable description of the invention.

The terms "a"/"an", as used herein, are defined as one or more than one. The term plurality, as used herein, is defined as two or more than two. The term another, as used herein, is defined as at least a second or more. The terms including and/or having, as used herein, are defined as comprising (i.e., open language, not excluding other elements or steps).

The mere fact that certain measures are recited in mutually different dependent claims does not indicate that a combination of these measures cannot be used to advantage.

All reaction conditions are under atmospheric pressure, unless otherwise indicated.

### Brief description of the drawings

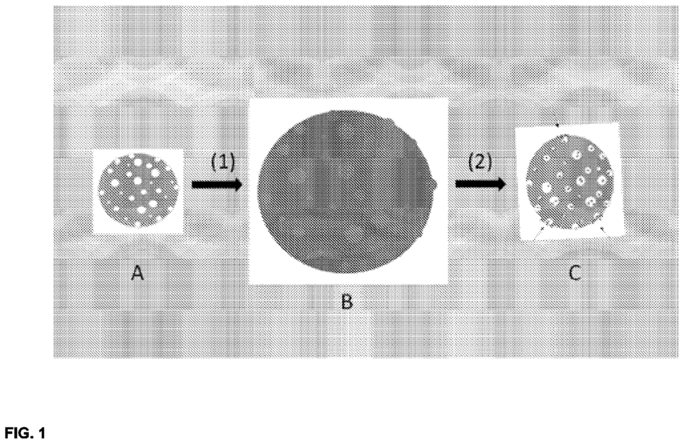

Figure 1 is a Schematic representation of the swelling/vacuum method for charging the porous embolic microspheres of this invention, with one, two, or more drugs (e.g. cytostatic drugs and/or anti-angiogenic drugs).
Figure 2 depicts scanning electron micrographs of the porous microspheres prior to drug loading. A, B, C, and D correspond to different magnifications. Scale bars in A, B, C, and D, are 100, 50, 10 and 30 micrometer, respectively.

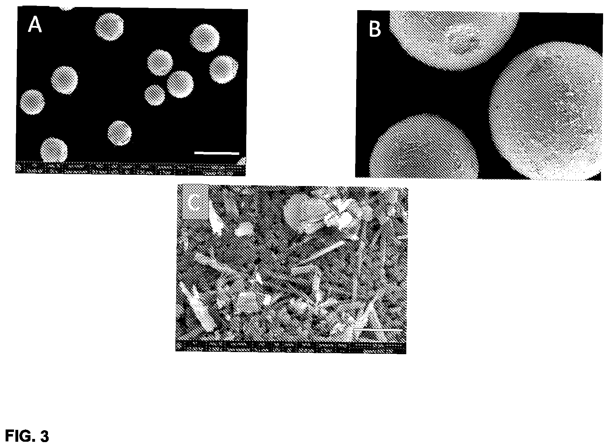

Figure 3 comprises scanning electron images of porous embolic microspheres according to the invention, loaded with the drug dipyridamole. A shows a number of drug-loaded microspheres at low magnification (scale bar in A is 500 micron). B shows an expansion of a region in A. C zooms in at the surface of these drug-loaded microspheres; the scale bar in C is 10 micron. In C, crystals of the drug are clearly visible at the surface of the microsphere. The crystals physically adhere to the microsphere since they are connected with other crystals residing in the interior pores of the microsphere.

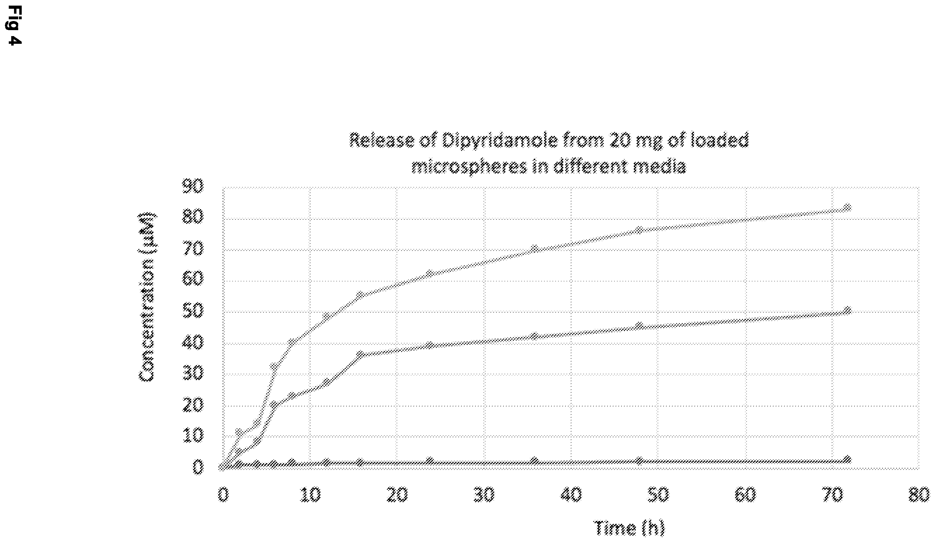

Figure 4 demonstrates release profiles of dipyridamole from charged embolic microspheres (20 mg) in vitro, measured at 37 deg. C. Three different media were used: Phosphate-buffered saline (PBS, lowest curve), PBS containing 5 % ethanol (middle curve), and PBS containing 10 % ethanol (highest curve). Evidently, release is accelerated by the presence of ethanol, which is a much better solvent for dipyridamole than water and other aqueous media (like PBS). In all cases, release continues after 3 days, which reveals that a mechanism for sustained release is operative.

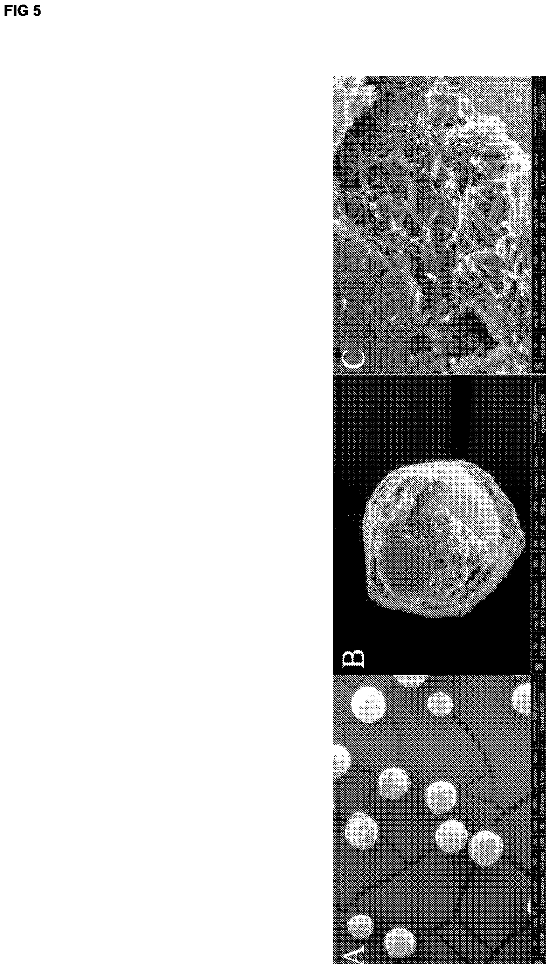

Figure 5 Scanning electron microscopy of the embolic microspheres which were charged with dipyridamole. A: overview, showing that the overall morphology of the microspheres has changed due to the presence of the drug at the surface. B. Enlarged view of one of the dipyridamole-loaded particles, clearly showing the drug at the surface. The adherence of the drug is due to the fact that drug crystals at the surface are connected with crystals residing inside the cavities in the interior of the microspheres. C. Further enlarged view showing needle-shaped crystals of the drug that is exposed at the particle's surface.

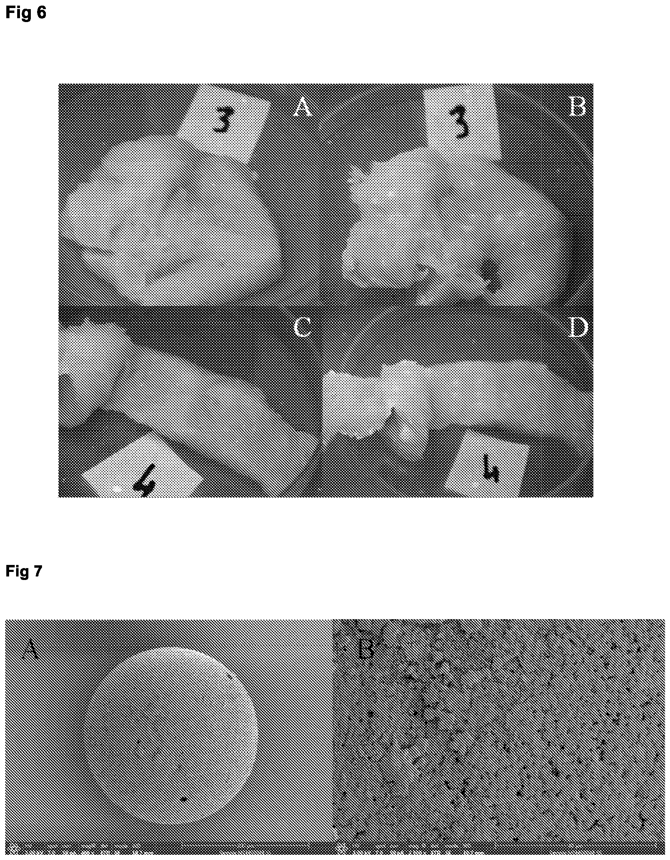

Figure 6 Demonstration of the occurrence of drug release from the embolic microspheres shown in Figure 5; i.e. these particles were loaded with dipyridamole. The particles were placed on a surface consisting of boneless chicken (A and B), or boneless pork (C and D). It was assumed that this soft-tissue environment more or less resembles the interior of the tumor. All images were taken under UV light, i.e. the particles then appear as green-fluorescent clear spots. A shows the presence of the particles immediately after. Note that the green spots are sharp and well-defined. The flap on the left side in Fig A was folded over the microspheres and the specimen was left untouched for 24 h (room temperature). Then, the flap was folded back, and image B was taken. Clearly, release of the dipyridamole has occurred: the green spots have expanded, AND green spots are present on the surface that was folded back. C and D show analogous results, now for the experiment with boneless pork.
Figure 7 Scanning electron micrograph of a porous/cavitated microsphere according to this invention, that was incubated with saturated NaCl solution. The particles were first submerged in the salt solution, and then a vacuum was applied. This implies that virtually all air residing in the cavities was evacuated. Then, when the vacuum is released, the salt solution is forced into the cavities in the particle's interior. Lyophilization produced the microspheres as shown in Extra Figure 4. Note that the surface is much less porous now. Also, salt crystals reside at the surface. Their adherence is explained by their physical connection with salt crystals that reside inside the particle.

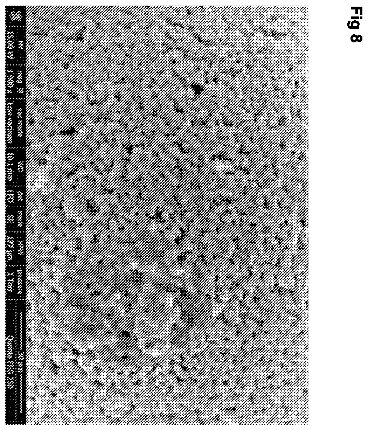

Figure 8 Close up of the porous/cavitated surface structure of the embolic microsphere particles of this invention. Note that the openings at the surface have a diameter around 3 micrometer. This agrees with the size of the silver particles that were used in the preparation. We noticed that using larger metal particles leads to structural weakness of the microspheres, which may then get damaged during size-sorting (sieving). Use of smaller particles is not optimal, since transport of matter during loading is experiencing much more resistance if the openings are of sub-micron size.

### Examples

### Example 1: Preparation of radiopaque, porous microspheres, method 1.

A solution was prepared containing 14.45 g of NaCl, 2 g of MgCl₂ × 8H₂O and 70 ml of water in a 250 ml round bottom flask. This was stirred at 450 rpm in a hot oil bath (87°C) for approximately 15 minutes. 0.79 g of NaOH pellets were weighed and 15 ml of water was added. This solution was swirled until the pellets were dissolved. This solution was added slowly to the mixture in the 250 ml round bottom flask and stirred for 15 minutes to precipitate Mg(OH)₂. In a 100ml round bottom flask 10.8 g of toluene + PMMA solution was weighed (made by mixing 7.56g of toluene with 3.24 g of PMMA and letting this stir overnight to create a uniform, clear, viscous mixture). In another 100ml round bottom flask 4.6 g of 4IEMA (15% iodine) was weighed and 6.2 g of HEMA was added to this. The 4IEMA was dissolved using the warmth of hand palms. 0.36 ml of TEGDMA and 0.776 ml of Trignox were added to the HEMA + 4IEMA solution, which was mixed by swirling the round bottom flask. The contents of the flask containing the HEMA + 4IEMA was added to the flask containing PMMA and toluene. This was mixed extensively by swirling the round bottom flask. A glass pipette was used to add this solution drop wise to the contents of the 250 ml round bottom flask (containing precipitated Mg(OH)₂). This solution was stirred at 350 rpm for 4 - 5 hours. Subsequently stirring was stopped and the solution was cooled to room temperature. Microspheres accumulated at the bottom of the flask. Solvent was decanted and the microspheres were extensively washed first with water and then with acetone. Toluene was added to the washed spheres and soaked overnight to extract PMMA from the spheres to create pores. The toluene was decanted from the spheres and acetone was added. This was soaked overnight. Acetone was removed from the microspheres and they were washed again with water. All the water was removed and the microspheres were freeze dried. The microspheres where then sorted into various size ranges using (manual) sieving. Two types of microspheres were synthesized using altered building block amounts.

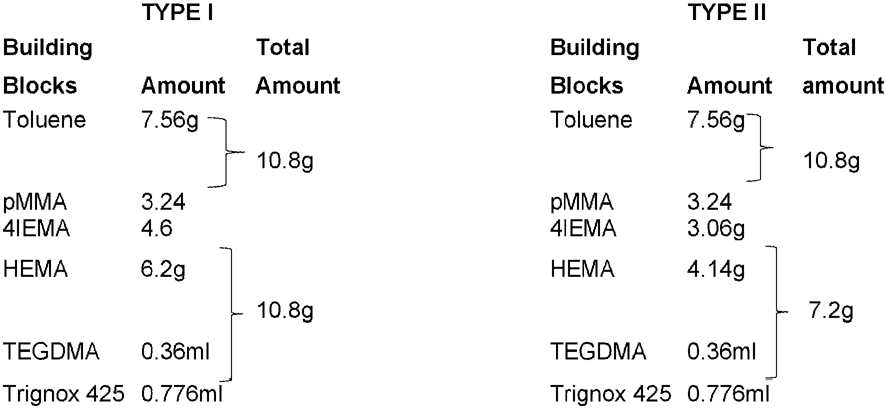

Figure 2 shows 4 representative images of the porous microspheres. A shows two comparable microspheres; the particle that is entirely visible has a diameter of approximately 120 micrometer, whereas the other microsphere (partly visible) is larger (diameter of approximately 200 micrometer). The scale bar in A (bottom, right) is 100 micrometer long. B is a magnification of the smaller microsphere of A. The scale bar in B (bottom right) is 50 micron. B illustrates the porous nature of the microsphere. C and D are magnified images of another analogous porous microsphere. In this case, larger pores at the microspheres' surface are encountered. The larger pore in C has a diameter of approximately 10 micron (scale bar in C (lover left) is 10 micron). D shows four similar larger "holes" at the surface. These larger pores can have significant importance for drug loading. Not only will they contain a relatively large amount of drug, they also play a role in stabilizing drug crystals which are deposited at the periphery of the microspheres (vide infra).

### Example 2: Preparation of radiopaque, porous microspheres, method 2.

First, an aqueous solution of poly(vinylalcohol) (PVA), poly(ethyleneglycol) (PEG) and poly(N-vinyl-pyrrollidone) (PVP) was made. PVA (63.0 g; Sigma-Aldrich), PEG (48.6 g; Sigma-Aldrich) and PVP (24 g; Acros) were dissolved in demineralized water (1800 mL); prolonged heating and stirring were required.

Secondly, a solution of poly(methylmethacrylate) (PMMA; 2.5 g) in methylmethacrylate (MMA, 20 g) was made.

Thirdly, a reactive cocktail was prepared, consisting of:
- The PMMA/MMA solution (4.57 g)
- 4-lodobenzoylmethacrylate (4IEMA) (3.19 g)
- Hydroxyethylmethacrylate (HEMA) (0.5 g)
- Crosslinker triethyleneglycoldimethacrylate (TEGDMA) (1.70 g)
- Trigonox (radical initiator, 0.88 g)

A 1000 mL round bottom flask was immersed in hot oil (100 °C). The temperature of the oil was controlled and remained in the interval 99.5 - 100.5 °C. The aqueous polymer solution (400 mL) was transferred into the round-bottom flask, and a magnetic stirring bar was added. The stirring speed was set at 500 rpm. The mixture was left for minimally 1h, to allow the contents of the flask to warm. At this stage, the reactive cocktail was mixed with 1 gram of silver microparticles (dimensions: 2 - 3.5 micrometer). The mixture was shaken vigorously in order to disperse the silver particles. The mixture was then added quickly to the hot stirred aqueous polymer solution.

The mixture was left for 3h. Then, heating was stopped. Stirring was continued. After several hours, stirring was stopped as well, and the oil bath was removed. Precipitation of microspheres was noted. The supernatant was discarded, and the microspheres were washed repeatedly with water. This provided microspheres with silver particles embedded in them. Microspheres of different sizes were obtained (as evidenced by light microscopic analysis). The diameter varied between 10 and 600 micrometer. Microscopic analysis also confirmed the presence of silver particles on and inside of the microspheres.

Subsequently, the microspheres were incubated and magnetically stirred in nitric acid (1M, Aldrich) at 75 °C. This led to dissolution of the silver, leaving micrometer-sized pores at the sites where silver particles sat first. The reaction dissolved all silver particles, irrespective of their position on or inside the microspheres. After 18 h, heating was stopped, and the flask and its contents were allowed to cool. The now porous microspheres were repeatedly washed with water and allowed to dry. Subsequently, the porous microspheres were size-sorted by sieving.

The experiment is repeated with solid PMMA particles of 2-4 micrometer. After formation of the microspheres, the microspheres are suspended in toluene overnight under agitation. The eluent is removed by filtering and the microspheres are dried. Microscopic inspection shows the formation of pores.

### Example 3: Loading porous microspheres with the drug Sorafenib

1. A stock solution of 200 mg sorafenib (free base) in DMSO (1.00 ml) was prepared..
2. Porous microspheres (200 mg, diameter range 100 - 300 micrometer) were weighed into a polypropylene centrifuge tube (10 ml).
3. The drug solution of step 1. was transferred into the centrifugation tube of step 2. The solution and the microspheres were mixed carefully and left to stand for 30 min.
4. Then, 5 ml of demineralized water was added. The tube was closed and shaken. Precipitation of the drug in the DMSO-water mixture was noted.
5. The supernatant was removed carefully (pipet), and new water (5ml) was added. Shaking was repeated.
6. Step 5 was repeated 4 times, and it was clear that the supernatant no longer contained drug crystals.
7. The tube was filled with a 0.1 % solution of sodiumdodecyl sulfate in water, and left to stand for 24 h.
8. The microspheres were transferred to a filter paper and left to dry overnight.
9. The dry microspheres were sieved (sieve with 100 micrometer mesh), to remove unbound and loosely bound drug crystals.
10. The microspheres on the sieve were collected and stored in a dark glass vial (-20 °C).

Figure 1A depicts a porous embolic microsphere that was produced according to the method as described above. The pores are distributed evenly throughout the body of each microsphere; the pores have different diameters, and some pores are found at the microsphere's surface. B depicts the equilibrium situation that is achieved upon incubation of the porous microsphere of A in a concentrated solution of one, two or more drugs in DMSO. Note that the microsphere as well as the pores have swollen considerably. Further, note that the pores are now filled with the drug solution, and that the body of the microsphere is also saturated with DMSO. Swelling up to equilibrium situation takes generally 4 - 6 h. C depicts the situation that is reached after sudden incubation of B in large excess of water.

### Example 4: Loading porous microspheres with dipyridamole

Microspheres were loaded with the drug dipyridamole using a similar method as described in example 3 above. Dipyridamole is a non-toxic cardiovascular drug which is readily available and which has solubility properties that closely resemble those of many cytostatic and anti-angiogenic agents (high solubility in DMSO, low solubility in water). Another particular advantage of dipyridamole is its strong UV absorbance and fluorescence, which enables visualization and qualitative and quantitative analysis of the drug release process.

Figure 3 indicates that drug crystals (needles in this case) are found at the particle's surface. Some of the needles protrude into the porous structure of the microsphere, thus providing a mechanism of physical binding to the particle's surface. It was observed that the attachment is robust and strong. For example, the attached crystals survived a sieving treatment which was executed to separate the drug-loaded microspheres from unbound and loosely bound drug crystals.

The porous microspheres, loaded with dipyridamole were subsequently incubated in ethanol (15 mg in 5 mL). Release of dipyridamole was clearly observed, as the supernatant turned yellow. The microspheres were left to stand for 24 with occasional shaking. Then, the supernatant was analyzed spectrophotometrically, to determine the concentration of dipyridamole. From that number, the loading of the microspheres was calculated.

Repeated experiments showed that the loading of the microspheres is 17 +/- 3 mass %. This implies that a substantial amount of the drug will be placed inside the tumor. For instance, if 200 mg of microspheres (a typical quantity) would be used in the treatment of a HCC, approximately 34 mg would be transported into the tumor to be released there.

Repetitive loading of the porous microspheres leads to increased load of active compound in the microspheres: a second round of loading increased the amount of dipyridamole drug included in the microsphere and pores thereof to 24 +/- 3 mass %. Triple loading yielded a load of 29 +/- 4 mass %, drawn on the total mass of the porous microsphere. This is significantly higher than the regular load of a comparative microsphere that does not have pores or cavities. Release profiles of porous microspheres having such high loads are characterized by a high initial burst from the therapeutic agent deposited on the surface, followed by a longer lasting sustained release from the pores and impregnation of the polymeric microspheres.

## Claims

1. Method of forming injectable polymeric microspheres loaded with therapeutic agent, comprising:
a. exposing porous polymeric microspheres to an organic solvent comprising a dissolved therapeutic agent thereby creating microspheres loaded with therapeutic agent,
b. separating the microspheres loaded with therapeutic agent from the organic solvent,
c. washing the microspheres loaded with therapeutic agent with water, and
d. drying the washed microspheres, preferably wherein the porous polymeric microspheres have pores having a size of between 2 and 50 micrometers, more preferably between 3 and 8 micrometers, even more preferably between 5 and 10 micrometers.

2. Method according to claim 1, wherein the organic solvent is at least partially miscible with water at 20 °C, preferably wherein the organic solvent is fully miscible with water at 20 °C, and/or
wherein the concentration of the therapeutic agent in the organic solvent is at least 20 mg/ml at 20 °C, preferably wherein the concentration of the therapeutic agent in the organic solvent is at least 100 mg/ml at 20 °C, more preferably at least 200 mg/ml at 20 °C, and/or
wherein the concentration of the therapeutic agent in the organic solvent is at most 800 mg/ml at 20 °C, preferably at most 500 mg/ml at 20 °C.

3. Method according to claim 1 or 2, wherein the solubility of the therapeutic agent in water is between 5 and 1000 mg/L at 20 °C, preferably wherein the solubility of the therapeutic agent in water is between 5 and 100 mg/L at 20 °C, more preferably between 10 and 50 mg/L at 20 °C, and/or
wherein the porous polymeric microspheres are swellable in the organic solvent to a swelling ratio Q, defined by the weight of the swollen particles divided by the weight of the dry particles, of about 1 - 100, preferably of 2 - 20, more preferably of 5 - 10.

4. Method according to any one of the preceding claims, wherein in step a. a reduced pressure is applied, and/or
wherein the solvent comprises multiple dissolved therapeutic agents, and/or
wherein the organic solvent is chosen from the group consisting of acetaldehyde, acetic acid, acetone, acetonitrile, 1,2-butanediol, 1,3-butanediol, 1,4-butanediol, 2-butoxyethanol, butyric acid, diethanolamine, diethylenetriamine, dimethylformamide, dimethoxyethane, dimethyl sulfoxide, 1,4-dioxane, ethanol, ethylamine, ethylene glycol, formic acid, furfuryl alcohol, glycerol, methanol, methyl diethanolamine, methyl isocyanide, N-methyl-2-pyrrolidinone, 1-propanol, 1,3-propanediol, 1,5-pentanediol, 2-propanol, propanoic acid, propylene glycol, pyridine, tetrahydrofuran, triethylene glycol, hexamethylphosphoramide, and/or mixtures thereof, preferably
wherein the organic solvent is chosen from the group consisting of dimethyl sulfoxide, hexamethylphosphoramide, dimethyl formamide, 1,2-butanediol, 1,3-butanediol, 1,4-butanediol, 2-butoxyethanol, ethanol, ethylene glycol, furfuryl alcohol, glycerol, methanol, 1-propanol, 1,3-propanediol, 1,5-pentanediol, 2-propanol, and/or mixtures thereof, more preferably
wherein the organic solvent is dimethyl sulfoxide.

5. Method according to any one of the preceding claims, wherein the therapeutic agent is an anti-cancer drug and/or an anti-angiogenic drug, preferably
wherein the therapeutic agent is chosen from the group consisting of sorafenib, irinotecan, cis-platin, paclitaxel, docetaxel, cabazitaxel, larotaxel, eribulin, ixabepilone, vinflumine, peretinoin, orantinib, brivanib, sunitinib, briganib, erlotinib, lenvatinib, crizotinib, vandetanib and/or combinations thereof, more preferably
wherein the therapeutic agent is chosen from the group consisting of sorafenib, paclitaxel, docetaxel, cabazitaxel, larotaxel, eribulin, ixabepilone, peretinoin, orantinib, brivanib, sunitinib, briganib, erlotinib, lenvatinib, crizotinib, vandetanib, and/or combinations thereof, most preferably
wherein the therapeutic agent is sorafenib.

6. Method according to any one of the preceding claims, wherein the porous polymeric microspheres are cross-linked, preferably wherein the cross-linked porous polymeric microspheres are internally cross-linked by a cross-linking agent, wherein the cross-linking agent preferably comprises at least two acrylate or methacrylate groups, more preferably wherein the crosslinking agent is triethylene glycol dimethacrylate (TEGDMA), even more preferably wherein the internally cross-linked porous polymeric microspheres comprise between 5 and 30 w% of TEGDMA, most preferably between 10 and 20 w% TEGDMA.

7. Method according to any one of the preceding claims, wherein the porous polymeric microspheres are hydrophilic, preferably wherein the porous polymeric microspheres comprise methacrylic monomer units, more preferably wherein the porous polymeric microspheres comprise methyl methacrylate (MMA), even more preferably wherein the porous polymeric microspheres comprise between 20 and 60 w% of MMA, most preferably between 30 and 50 w% of MMA, and/or
wherein the porous polymeric microspheres comprise hydroxyethyl methacrylate (HEMA), preferably wherein the porous polymeric microspheres comprise between 20 and 60 w% of HEMA, more preferably between 30 and 50 w% of HEMA.

8. Method according to any one of the preceding claims, wherein the porous polymeric microspheres are intrinsically radiopaque, preferably wherein the porous polymeric microspheres comprise iodine, more preferably wherein the porous polymeric microspheres comprise 2-[4-iodobenzoyloxy]-ethyl methacrylate (4IEMA), even more preferably wherein the porous polymeric microspheres comprise between 5 and 30 w% of 4IEMA, most preferably between 10 and 20 w%.

9. Method according to any one of the preceding claims, wherein the porous polymeric microspheres comprise a copolymer of 4IEMA, MMA and HEMA, which copolymer is cross-linked with TEGDMA, preferably wherein the porous polymeric microspheres comprise a copolymer of between 10 and 20 w% of 4IEMA, between 30 and 50 w% of MMA, between 30 and 50 w% of HEMA, and between 10 and 20 w% of TEGDMA, the total adding up to 100w%.

10. Method according to any one of the preceding claims, wherein the pores have been obtained by addition of solid particles during synthesis of the microspheres, and subsequent dissolution of these solid particles, preferably wherein the solid particle is a non-noble metal or a polymer, and/or
wherein the pores are distributed throughout the entire volume of the porous polymeric microspheres, preferably wherein the pores have been obtained by addition of silver particles during synthesis of the microspheres, and subsequent dissolution of these silver particles.

11. Injectable polymeric microspheres loaded with therapeutic agent obtainable by the method of any one of claims 1 - 10, preferably wherein the injectable polymeric microspheres have a diameter in the range of from 1 - 1000 µm, more preferably of from 1 - 200 µm, even more preferably of from 50 - 100 µm, and/or
wherein the injectable polymeric microspheres do not swell to more than 400 % of their dry size in a physiological fluid, preferably not to more than 200 %, and/or
wherein the injectable polymeric microspheres loaded with therapeutic agent comprise at least 10 w%, preferably at least 12 w%, more preferably at least 14 w% of therapeutic agent, and/or
wherein the injectable polymeric microspheres comprise crystals of therapeutic agent on the outer surface of the microspheres interconnected with crystals of therapeutic agent inside the pores.

12. Kit comprising a container with injectable polymeric microspheres loaded with therapeutic agent according to claim 11, a container with a pharmaceutically injectable liquid, and optionally, a container with contrast agent.

13. Pharmaceutical composition comprising injectable polymeric microspheres loaded with therapeutic agent according to claim 11 and preferably one or more of a pharmaceutically acceptable diluent, vehicle, and/or excipient.

14. Injectable polymeric microspheres according to claim 11 for use as a medicament, preferably for the embolization treatment of tumours.

15. Pharmaceutical composition according to claim 13 for use as a medicament, preferably for the embolization treatment of tumours.

## Patentansprüche

1. Verfahren zur Bildung von injizierbaren polymeren Mikrokugeln, die mit einem therapeutischen Wirkstoff beladen sind, umfassend:
a) Aussetzen poröser polymerer Mikrokugeln einem organischen Lösungsmittel, das einen gelösten therapeutischen Wirkstoff umfasst, wodurch mit therapeutischem Wirkstoff beladene Mikrokugeln entstehen,
b) Trennen der mit dem therapeutischen Wirkstoff beladenen Mikrokügelchen vom organischen Lösungsmittel,
c) Waschen der mit einem therapeutischen Wirkstoff beladenen Mikrokugeln mit Wasser, und
d) Trocknen der gewaschenen Mikrokugeln, bevorzugt wobei die porösen polymeren Mikrokugeln Poren mit einer Größe zwischen 2 und 50 Mikrometern, bevorzugter zwischen 3 und 8 Mikrometern, noch bevorzugter zwischen 5 und 10 Mikrometern aufweisen.

2. Verfahren nach Anspruch 1, wobei das organische Lösungsmittel bei 20°C zumindest teilweise mit Wasser mischbar ist, bevorzugt wobei das organische Lösungsmittel bei 20°C vollständig mit Wasser mischbar ist, und/oder
wobei die Konzentration des therapeutischen Wirkstoffs in dem organischen Lösungsmittel mindestens 20 mg/ml bei 20°C beträgt, bevorzugt wobei die Konzentration des therapeutischen Wirkstoffa in dem organischen Lösungsmittel mindestens 100 mg/ml bei 20°C, bevorzugter mindestens 200 mg/ml bei 20°C beträgt, und/oder
wobei die Konzentration des therapeutischen Wirkstoffs in dem organischen Lösungsmittel höchstens 800 mg/ml bei 20°C, bevorzugt höchstens 500 mg/ml bei 20°C beträgt.

3. Verfahren nach Anspruch 1 oder 2, wobei die Löslichkeit des therapeutischen Wirkstoffs in Wasser zwischen 5 und 1000 mg/l bei 20°C liegt, bevorzugt wobei die Löslichkeit des therapeutischen Wirkstoffs in Wasser zwischen 5 und 100 mg/l bei 20°C, bevorzugter zwischen 10 und 50 mg/l bei 20°C liegt, und/oder
wobei die porösen polymeren Mikrokugeln in dem organischen Lösungsmittel bis zu einem Quellverhältnis Q, definiert durch das Gewicht der gequollenen Partikel dividiert durch das Gewicht der trockenen Partikel, von etwa 1-100, bevorzugt von 2-20, bevorzugter von 5-10, quellbar sind.

4. Verfahren nach einem der vorstehenden Ansprüche, wobei in Schritt a. ein verminderter Druck aufgebracht wird, und/oder
wobei das Lösungsmittel mehrere gelöste therapeutische Wirkstoffe umfasst, und/oder
wobei das organische Lösungsmittel ausgewählt ist aus der Gruppe bestehend aus Acetaldehyd, Essigsäure, Aceton, Acetonitril, 1,2-Butandiol, 1,3-Butandiol, 1,4-Butandiol, 2-Butoxyethanol, Buttersäure, Diethanolamin, Diethylentriamin, Dimethylformamid, Dimethoxyethan, Dimethylsulfoxid, 1,4-Dioxan, Ethanol, Ethylamin, Ethylenglykol, Ameisensäure, Furfurylalkohol, Glycerin, Methanol, Methyldiethanolamin, Methylisocyanid, N-Methyl-2-pyrrolidinon, 1-Propanol, 1,3-Propandiol, 1,5-Pentandiol, 2-Propanol, Propansäure, Propylenglykol, Pyridin, Tetrahydrofuran, Triethylenglykol, Hexamethylphosphoramid und/oder Mischungen davon, bevorzugt
wobei das organische Lösungsmittel ausgewählt ist aus der Gruppe bestehend aus Dimethylsulfoxid, Hexamethylphosphoramid, Dimethylformamid, 1,2-Butandiol, 1,3-Butandiol, 1,4-Butandiol, 2-Butoxyethanol, Ethanol, Ethylenglykol, Furfurylalkohol, Glycerin, Methanol, 1-Propanol, 1,3-Propandiol, 1,5-Pentandiol, 2-Propanol und/oder Gemischen davon, bevorzugter
wobei das organische Lösungsmittel Dimethylsulfoxid ist.

5. Verfahren nach einem der vorstehenden Ansprüche, wobei es sich bei dem therapeutischen Wirkstoff um ein Anti-Krebs-Arzneimittel und/oder ein anti-angiogenes Arzneimittel handelt, bevorzugt
wobei der therapeutische Wirkstoff ausgewählt ist aus der Gruppe bestehend aus Sorafenib, Irinotecan, Cis-Platin, Paclitaxel, Docetaxel, Cabazitaxel, Larotaxel, Eribulin, Ixabepilon, Vinflumin, Peretinoin, Orantinib, Brivanib, Sunitinib, Briganib, Erlotinib, Lenvatinib, Crizotinib, Vandetanib und/oder Kombinationen davon, bevorzugter
wobei der therapeutische Wirkstoff ausgewählt ist aus der Gruppe bestehend aus Sorafenib, Paclitaxel, Docetaxel, Cabazitaxel, Larotaxel, Eribulin, Ixabepilon, Peretinoin, Orantinib, Brivanib, Sunitinib, Briganib, Erlotinib, Lenvatinib, Crizotinib, Vandetanib und/oder Kombinationen davon, am bevorzugtesten
wobei der therapeutische Wirkstoff Sorafenib ist.

6. Verfahren nach einem der vorstehenden Ansprüche, wobei die porösen polymeren Mikrokugeln vernetzt sind, bevorzugt wobei die vernetzten porösen polymeren Mikrokugeln intern durch einen Vernetzungswirkstoff vernetzt sind, wobei der Vernetzungswirkstoff bevorzugt mindestens zwei Acrylat- oder Methacrylatgruppen umfasst, bevorzugter, wobei es sich bei dem Vernetzungswirkstoff um Triethylenglykoldimethacrylat (TEGDMA) handelt, noch bevorzugter, wobei die intern vernetzten porösen polymeren Mikrokugeln zwischen 5 und 30 Gew.-% TEGDMA umfassen, am bevorzugtesten zwischen 10 und 20 Gew.-% TEGDMA.

7. Verfahren nach einem der vorstehenden Ansprüche, wobei die porösen polymeren Mikrokugeln hydrophil sind, bevorzugt wobei die porösen polymeren Mikrokugeln Methacrylmonomereinheiten umfassen, bevorzugter wobei die porösen polymeren Mikrokugeln Methylmethacrylat (MMA) umfassen, noch bevorzugter wobei die porösen polymeren Mikrokugeln zwischen 20 und 60 Gew.-% MMA umfassen, am bevorzugtesten zwischen 30 und 50 Gew.-% MMA, und/oder
wobei die porösen polymeren Mikrokugeln Hydroxyethylmethacrylat (HEMA) umfassen, bevorzugt wobei die porösen polymeren Mikrokugeln zwischen 20 und 60 Gew.-% HEMA umfassen, noch bevorzugter zwischen 30 und 50 Gew.-% HEMA.

8. Verfahren nach einem der vorstehenden Ansprüche, wobei die porösen polymeren Mikrokugeln intrinsisch röntgenopak sind, bevorzugt, wobei die porösen polymeren Mikrokugeln Iod umfassen, bevorzugter, wobei die porösen polymeren Mikrokugeln 2-[4-lodbenzoyloxy]-ethylmethacrylat (4IEMA) umfassen, noch bevorzugter, wobei die porösen polymeren Mikrokugeln zwischen 5 und 30 Gew.-% 4IEMA umfassen, am bevorzugtesten zwischen 10 und 20 Gew.-%.

9. Verfahren nach einem der vorstehenden Ansprüche, wobei die porösen polymeren Mikrokugeln ein Copolymer aus 4IEMA, MMA und HEMA umfassen, wobei das Copolymer mit TEGDMA vernetzt ist, bevorzugt wobei die porösen polymeren Mikrokugeln ein Copolymer aus zwischen 10 und 20 Gew.-% 4IEMA, zwischen 30 und 50 Gew.-% MMA, zwischen 30 und 50 Gew.-% HEMA und zwischen 10 und 20 Gew.-% TEGDMA umfassen, wobei die Gesamtsumme bis zu 100 Gew.-% beträgt.

10. Verfahren nach einem der vorstehenden Ansprüche, wobei die Poren durch Zugabe von Feststoffpartikeln während der Synthese der Mikrokugeln und anschließende Auflösung dieser Feststoffpartikel erhalten wurden, bevorzugt wobei das Feststoffpartikel ein unedles Metall oder ein Polymer ist, und/oder
wobei die Poren über das gesamte Volumen der porösen polymeren Mikrokugeln verteilt sind, bevorzugt wobei die Poren durch Zugabe von Silberpartikeln während der Synthese der Mikrokugeln und anschließendes Auflösen dieser Silberpartikel erhalten wurden.

11. Injizierbare polymere Mikrokugeln, die mit einem therapeutischen Wirkstoff beladen sind, erhältlich nach dem Verfahren nach einem der Ansprüche 1-10, bevorzugt wobei die injizierbaren polymeren Mikrokugeln einen Durchmesser im Bereich von 1-1000 µm, bevorzugter von 1-200 µm, noch bevorzugter von 50-100 µm aufweisen, und/oder
wobei die injizierbaren polymeren Mikrokügelchen in einem physiologischen Fluid nicht auf mehr als 400 % ihrer Trockengröße anschwellen, bevorzugt nicht auf mehr als 200 %, und/oder
wobei die injizierbaren polymeren Mikrokügelchen, die mit therapeutischem Wirkstoff beladen sind, mindestens 10 Gew.-%, bevorzugt mindestens 12 Gew.-%, bevorzugter mindestens 14 Gew.-% therapeutischen Wirkstoff umfassen, und/oder
wobei die injizierbaren polymeren Mikrokugeln Kristalle des therapeutischen Wirkstoffs auf der Außenoberfläche der Mikrokugeln umfassen, die mit Kristallen des therapeutischen Wirkstoffs im Inneren der Poren verbunden sind.

12. Kit, umfassend einen Behälter mit injizierbaren polymeren Mikrokügelchen, die mit einem therapeutischen Wirkstoff nach Anspruch 11 beladen sind, einen Behälter mit einer pharmazeutisch injizierbaren Flüssigkeit und wahlweise einen Behälter mit einem Kontrastmittel.

13. Pharmazeutische Zusammensetzung, umfassend injizierbare polymere Mikrokügelchen, die mit einem therapeutischen Wirkstoff nach Anspruch 11 beladen sind, und bevorzugt ein oder mehrere pharmazeutisch annehmbare Verdünnungsmittel, Träger und/oder Hilfsstoffe.

14. Injizierbare polymere Mikrokugeln nach Anspruch 11 zur Verwendung als Arzneimittel, bevorzugt für die Embolisationsbehandlung von Tumoren.

15. Pharmazeutische Zusammensetzung nach Anspruch 13 zur Verwendung als Arzneimittel, bevorzugt für die Embolisationsbehandlung von Tumoren.

## Revendications

1. Procédé de formation de microsphères polymères injectables chargées d'un agent thérapeutique, comprenant les étapes consistant à :
a. l'exposition de microsphères polymères poreuses à un solvant organique comprenant un agent thérapeutique dissous, créant ainsi des microsphères chargées d'agent thérapeutique,
b. la séparation des microsphères chargées d'agent thérapeutique du solvant organique,
c. le lavage des microsphères chargées d'agent thérapeutique avec de l'eau, et
d. le séchage des microsphères lavées, de préférence dans lequel les microsphères polymères poreuses ont des pores ayant une taille comprise entre 2 et 50 micromètres, plus préférablement entre 3 et 8 micromètres, encore plus préférablement entre 5 et 10 micromètres.

2. Procédé selon la revendication 1, dans lequel le solvant organique est au moins partiellement miscible à l'eau à 20°C, de préférence dans lequel le solvant organique est totalement miscible à l'eau à 20°C, et/ou
dans lequel la concentration de l'agent thérapeutique dans le solvant organique est d'au moins 20 mg/ml à 20°C, de préférence dans lequel la concentration de l'agent thérapeutique dans le solvant organique est d'au moins 100 mg/ml à 20°C, et plus préférablement d'au moins 200 mg/ml à 20°C, et/ou dans lequel la concentration de l'agent thérapeutique dans le solvant organique est d'au plus 800 mg/ml à 20°C, de préférence d'au plus 500 mg/ml à 20°C.

3. Procédé selon la revendication 1 ou 2, dans lequel la solubilité de l'agent thérapeutique dans l'eau est comprise entre 5 et 1000 mg/L à 20°C, de préférence dans lequel la solubilité de l'agent thérapeutique dans l'eau est comprise entre 5 et 100 mg/L à 20°C, plus préférablement entre 10 et 50 mg/L à 20°C, et/ou
dans lequel les microsphères polymères poreuses sont gonflables dans le solvant organique à un rapport de gonflement Q, défini par le poids des particules gonflées divisé par le poids des particules sèches, d'environ 1 à 100, de préférence de 2 à 20, plus préférablement de 5 à 10.

4. Procédé selon l'une quelconque des revendications précédentes, dans lequel à l'étape a. une pression réduite est appliquée, et/ou
dans lequel le solvant comprend plusieurs agents thérapeutiques dissous, et/ou
dans lequel le solvant organique est choisi dans le groupe constitué par l'acétaldéhyde, l'acide acétique, l'acétone, l'acétonitrile, le 1,2-butanediol, le 1,3-butanediol, le 1,4-butanediol, le 2-butoxyéthanol, l'acide butyrique, la diéthanolamine, la diéthylènetriamine, le diméthylformamide, le diméthoxyéthane, le diméthylsulfoxyde, le 1,4-dioxane, l'éthanol, l'éthylamine, l'éthylène glycol, l'acide formique, l'alcool furfurylique, le glycérol, le méthanol, la méthyldiéthanolamine, l'isocyanure de méthyle, la N-méthyl-2-pyrrolidinone, le 1-propanol, le 1,3-propanediol, le 1,5-pentanediol, le 2-propanol, l'acide propanoïque, le propylène glycol, la pyridine, le tétrahydrofurane, le triéthylène glycol, l'hexaméthylphosphoramide, et/ou leurs mélanges, de préférence
dans lequel le solvant organique est choisi dans le groupe constitué par le diméthylsulfoxyde, l'hexaméthylphosphoramide, le diméthylformamide, le 1,2-butanediol, le 1,3-butanediol, le 1,4-butanediol, le 2-butoxyéthanol, l'éthanol, l'éthylène glycol, l'alcool furfurylique, le glycérol, le méthanol, le 1-propanol, le 1,3-propanediol, le 1,5-pentanediol, le 2-propanol, et/ou leurs mélanges, plus préférablement
dans lequel le solvant organique est le diméthylsulfoxyde.

5. Procédé selon l'une quelconque des revendications précédentes, dans lequel l'agent thérapeutique est un médicament anticancéreux et/ou un médicament anti-angiogénique, de préférence
dans lequel l'agent thérapeutique est choisi dans le groupe constitué par le sorafénib, l'irinotécan, le cis-platine, le paclitaxel, le docétaxel, le cabazitaxel, le larotaxel, l'éribuline, l'ixabépilone, la vinflumine, la pérétinoïne, l'orantinib, le brivanib, le sunitinib, le briganib, l'erlotinib, le lenvatinib, le crizotinib, le vandétanib et/ou leurs combinaisons, plus préférablement dans lequel l'agent thérapeutique est choisi dans le groupe constitué par le sorafénib, le paclitaxel, le docétaxel, le cabazitaxel, le larotaxel, l'éribuline, l'ixabépilone, la pérétinoïne, l'orantinib, le brivanib, le sunitinib, le briganib, l'erlotinib, le lenvatinib, le crizotinib, le vandétanib, et/ou des combinaisons de ceux-ci, le plus préférablement
dans lequel l'agent thérapeutique est le sorafenib.

6. Procédé selon l'une quelconque des revendications précédentes, dans lequel les microsphères polymériques poreuses sont réticulées, de préférence dans lequel les microsphères polymériques poreuses réticulées sont réticulées de manière interne par un agent de réticulation, dans lequel l'agent de réticulation comprend de préférence au moins deux groupes acrylate ou méthacrylate, plus préférablement dans lequel l'agent de réticulation est le diméthacrylate de triéthylène glycol (TEGDMA), encore plus préférablement dans lequel les microsphères polymères poreuses réticulées intérieurement comprennent entre 5 et 30% en poids de TEGDMA, le plus préférablement entre 10 et 20% en poids de TEGDMA.

7. Procédé selon l'une quelconque des revendications précédentes, dans lequel les microsphères polymères poreuses sont hydrophiles, de préférence dans lequel les microsphères polymères poreuses comprennent des motifs monomères méthacryliques, plus préférablement dans lequel les microsphères polymères poreuses comprennent du méthacrylate de méthyle (MMA), encore plus préférablement, dans lequel les microsphères polymères poreuses comprennent entre 20 et 60% en poids de MMA, de préférence entre 30 et 50% en poids de MMA, et/ou
dans lequel les microsphères polymères poreuses comprennent du méthacrylate d'hydroxyéthyle (HEMA), de préférence dans lequel les microsphères polymères poreuses comprennent entre 20 et 60% en poids de HEMA, plus préférablement entre 30 et 50% en poids de HEMA.

8. Procédé selon l'une quelconque des revendications précédentes, dans lequel les microsphères polymères poreuses sont intrinsèquement radio-opaques, de préférence dans lequel les microsphères polymères poreuses comprennent de l'iode, plus préférentiellement dans lequel les microsphères polymères poreuses comprennent du méthacrylate de 2-[4-iodobenzoyloxy]-éthyle (4IEMA), encore plus préférentiellement dans lequel les microsphères polymères poreuses comprennent entre 5 et 30% en poids de 4IEMA, le plus préférablement entre 10 et 20% en poids.

9. Procédé selon l'une quelconque des revendications précédentes, dans lequel les microsphères polymères poreuses comprennent un copolymère de 4IEMA, MMA et HEMA, lequel copolymère est réticulé avec du TEGDMA, de préférence dans lequel les microsphères polymères poreuses comprennent un copolymère d'entre 10 et 20% en poids de 4IEMA, entre 30 et 50% en poids de MMA, entre 30 et 50% en poids de HEMA, et entre 10 et 20% en poids de TEGDMA, le total arrivant à 100% en poids.

10. Procédé selon l'une quelconque des revendications précédentes, dans lequel les pores ont été obtenus par addition de particules solides lors de la synthèse des microsphères, et dissolution ultérieure de ces particules solides, de préférence dans lequel la particule solide est un métal non noble ou un polymère, et/ou
dans lequel les pores sont distribués dans tout le volume des microsphères polymères poreuses, de préférence dans lequel les pores ont été obtenus par addition de particules d'argent pendant la synthèse des microsphères, et dissolution ultérieure de ces particules d'argent.

11. Microsphères polymères injectables chargées d'un agent thérapeutique pouvant être obtenues par le procédé de l'une quelconque des revendications 1 à 10, de préférence dans lesquelles les microsphères polymères injectables ont un diamètre dans la gamme de 1 à 1000 µm, plus préférablement de 1 à 200 µm, encore plus préférablement de 50 à 100 µm, et/ou dans lesquelles les microsphères polymères injectables ne gonflent pas à plus de 400% de leur taille sèche dans un fluide physiologique, de préférence pas à plus de 200%, et/ou dans lesquelles les microsphères polymères injectables chargées d'agent thérapeutique comprennent au moins 10% en poids, de préférence au moins 12% en poids, plus préférablement au moins 14% en poids d'agent thérapeutique, et/ou
dans lesquelles les microsphères polymères injectables comprennent des cristaux d'agent thérapeutique sur la surface extérieure des microsphères interconnectés avec des cristaux d'agent thérapeutique à l'intérieur des pores.

12. Kit comprenant un récipient avec des microsphères polymères injectables chargées d'agent thérapeutique selon la revendication 11, un récipient avec un liquide pharmaceutiquement injectable, et éventuellement, un récipient avec un agent de contraste.

13. Composition pharmaceutique, comprenant des microsphères polymères injectables chargées d'un agent thérapeutique selon la revendication 11 et de préférence un ou plusieurs diluants, véhicules et/ou excipients pharmaceutiquement acceptables.

14. Microsphères polymères injectables selon la revendication 11 pour une utilisation en tant que médicament, de préférence pour le traitement par embolisation de tumeurs.

15. Composition pharmaceutique selon la revendication 13 pour une utilisation en tant que médicament, de préférence pour le traitement par embolisation de tumeurs.
